# EUROPEAN PATENT SPECIFICATION

(11) **EP 2 566 549 B1**
(45) Date of publication and mention of the grant of the patent: **22.03.2017**
(21) Application number: 11720619.3
(22) Date of filing: 13.04.2011
(51) Int. Cl.: A61M 15/00

(54) **DRY POWDER INHALER MOUTHPIECE BUTTON**
TROCKENPULVERINHALATOR-MUNDSTÜCK
BOUTON D'EMBOUT BUCCAL POUR INHALATEUR À POUDRE SÈCHE

(30) Priority: 28.05.2010 TR 201004312; 20.04.2010 TR 201003091; 13.04.2010 TR 201002877
(43) Date of publication of application: 13.03.2013
(73) Proprietor: Sima Patent ve Lisanslama Hizmetleri Ltd.Sti., Esenler/Istanbul (TR)
(72) Inventor: Bilgic, Mahmut, Merter/Istanbul 34173 (TR)
(86) International application number: PCT/TR2011/000095
(87) International publication number: WO 2011/129795

(56) References cited:
- US-A1- 2005 005 934
- US-A1- 2005 172 964
- US-A1- 2006 196 504
- US-A1- 2009 078 252

## Description

### Field of Invention

The present invention relates to an inhaler suitable for delivering medicament in dry powder form used for respiratory diseases, particularly for asthma and chronic obstructive pulmonary disease (COPD). In addition, the present invention relates to an inhaler which comprises blister package suitable for carrying dry powder medicament and is used to realize an effective inhalation.

### Description of the Prior Art

Documents US 2009/0078252 A1 and US 2005/0172964 A1 disclose dry powder inhalers that are similar to that disclosed herein.

It is quite common to use inhalers for delivering medicaments utilized in the treatment and prophylaxis of respiratory diseases. Inhalation treatment is the most commonly preferred treatment method in these diseases as the inhalers provide ease of use; the medicaments have rapider onset of time resulting from local administration and they have fewer side effects. Various inhalers have been designed in order to provide effective and sufficient delivery of the medicaments used in the treatment of respiratory diseases, particularly in asthma and chronic obstructive pulmonary disease. These inhalers vary according to their operating mechanisms and the physical form of the medicament to be delivered.

In the inhalers used to deliver the medicaments in dry powder form, the medicament is carried in reservoirs, capsules or blisters packages. It is highly significant to deliver each dose to the patient with exact accuracy and preciseness since the required amounts of dry powder medicament dose for the inhalation is very low.

In an inhaler which is operated by a gear mechanism and comprises peelable blister strip package, one blister pocket is opened and the dry powder medicament contained in this blister pocket becomes ready for inhalation as a result of the blister package's, which is indexed in response to each actuation of the inhaler, being peeled by the mechanical component generally called the beak which is placed in the housing and has a sharp point.

However, one of the most significant problems encountered in inhalers comprising peelable blister packages is failure to deliver the sufficient amount of the active agent to the patient as the blister pocket is not completely opened in response to each actuation of the device. The fact that the lid sheet of the blister pocket is torn or broken off while it is being peeled away from the base sheet results in failure to open the blister pocket or failure to open it completely and causes some of the medicament in dry powder form to remain in the blister. As the active agents used in inhalation treatment have very strong effects, the amount of the active agent in one dose of medicament in dry powder form is quite low. Therefore, it is highly significant to deliver the required amount of the active agent to the patient's lungs. The fact that the blister cannot be opened completely and some dry powder medicament remains in the blister pocket in response to each actuation of the inhaler results in failure to provide the inhalation of the required amount of the active agent. When less than the required amount of the dry powder medicament is inhaled, an effective treatment cannot be achieved.

The fact that the lid sheet of the blister package indexed upon the actuation of the inhaler is broken off leads to a complete failure in inhalation of the medicament in dry powder form and the inhaler cannot be used again. Thus, the patient is left in a difficult situation as he cannot take the medicament during the crisis and the blister pockets remained unused in the device are wasted.

In addition, prevention of the medicament in dry powder form contained in the completely opened blister pocket to spill in the device during the inhalation of the patient contributes to delivery of required amount of active agent to the patient.

The inhalation device marketed under the trade mark Diskus® by GlaxoSmithKlein is one of the most well-known inhalers on the market. This device comprises blister strip package that is used to carry medicament in dry powder form and the gear mechanism of the device is operated by rotating the slide along a path. However, Diskus® inhaler may remain incapable to deliver the dry powder medicament to the patient.

The inventor has surprisingly found that the blister pocket can be completely opened without being torn or broken off and the sufficient amount of the dry powder medicament can inhaled in response to each actuation of the inhaler in the case that the shape and the position of the mechanical component which is generally called the beak and enables to peel the blister package in the inhaler comprising peelable blister package is adjusted.

In addition, the inventor has found that the position of the beak enabling to peel the blister package contributes to the inhalation of the sufficient amount of the dry powder medicament in the opened blister pocket during the inhalation and prevents the medicament in dry powder form to spill in the inhaler during the inhalation.

According to this, the present invention relates to an inhaler designed so as to enable the inhalation of sufficient amount of the active agent comprised in the dry powder medicament which is contained in the blister in response to each actuation of the inhaler comprising blister package.

### Summary of the Invention

The scope of the invention is as defined by the appended claims.

An inhaler suitable for delivery of the medicament in dry powder form according to the present invention comprising;
- a blister package composed of a plurality of blister pockets each of which comprises medicament in dry powder form and which are spaced at equal intervals;
- a beak which provides to peel the blister package
- a mouthpiece enabling the patient to inhale the medicament in dry powder form from the opened blister;
- a rotatable mouthpiece cover covering the mouthpiece;
- a gear mechanism enabling the blister package to be indexed and the medicament in dry powder form to become ready for inhalation;
- a housing situated between the upper housing member and the lower housing member in which the blister package and the gear mechanism are enclosed
is characterized in that the radius of the sharp point of the beak that is in contact with the lid sheet of the blister package indexed upon the actuation of the device is in the range of 0.05 to 1 mm.

In terms of providing ease of use to the patients, the inhaler pertaining to the present invention is preferably actuated by moving the rotatable mouthpiece cover. Actuation of the device causes the gear mechanism to index the blister package. The blister package is peeled by the beak while being indexed by the gear mechanism at the same time. The sharp point of the beak peels the lid sheet away from the base sheet of the blister package and provides to open one blister pocket in response to each actuation of the inhaler. The radius of the sharp point of the beak which is in contact with the lid sheet of the blister package plays a significant role in opening the blister pocket without tearing or breaking off the lid sheet. In more detail, the tearing or breaking off of the lid sheet can be prevented when the radius of the sharp point of the beak is in the range of 0.05 to 1 mm. In the case that the radius of the beak is at a lower value than this range, the lid sheet of the blister package may be torn or broken off. This may result in failure to open the blister pocket completely upon the actuation of the device or the inhaler may not be used again. In the case that the device cannot be used again, the unused blisters are wasted and the patient is left in a difficult situation. When the blister pocket cannot be opened completely, on the other hand, the intended therapeutic effect is not provided as the sufficient amount of the dry powder medicament in the blister pocket. In the case that radius of the sharp point the beak is at a higher value than the range of 0.05 to 1 mm, one blister pocket of the blister package that is indexed by the actuation of the device may not be opened completely. However, keeping the radius of the sharp point of the beak in the range of 0.05 to 1 mm, preferably in the range of 0.05 to 0.4 m, most preferably 0.05 to 0.3 mm provides a safe use of the inhaler by enabling the blister to be peeled completely in response to each actuation of the inhaler. Thus, the patient makes sure that one dose of dry powder medicament is ready for inhalation upon each actuation of the device.

In addition to this, one dose of dry powder medicament in the blister pocket opened upon the actuation of the device can be inhale on a sufficient amount in the case that the distance between the sharp point of the beak and the sharp point of the manifold is in the range of 0.005 to 0.5 mm, preferably 0.05 to 0.4 mm. Therefore, the desired therapeutic effect of the dry powder medicament is provided.

The inhaler pertaining to the present invention is an easy-grip and manual device which is appropriate for delivering medicament in dry powder form.

The housing of the device pertaining to the present invention has been designed such that each component of the blister package and the gear mechanism which have a significant role in enabling the device to work properly is situated accurately and works harmoniously. To this end, the housing is divided into several compartments. The used portion and the unused portion of the blister package are accommodated in separated compartments in order to prevent the medicament in dry powder form remained in the opened blister pocket to spill on the other components of the housing. Furthermore, the housing also comprises the beak which enables the blister package to be peeled and the manifold through which the dry powder medicament in the open blister passes before reaching the mouthpiece during the inhalation. In addition, the housing can be in any appropriate shape while it is preferably elliptic or circular.

The upper and the lower housing members interlock with each other and enclose the housing in order to keep the housing and the gear mechanism fixed together. The mouthpiece cover hiding the mouthpiece is rotated by being slid on the upper and lower housing members. The grids on the surface of the lower and the upper housing members provide an effective actuation by preventing the slipping of the finger while rotating the mouthpiece cover. The grids on the upper and the lower housing members provide an effective actuation by preventing the slips of the finger while rotating the mouthpiece cover. The upper and the lower housing members can be in any appropriate shape which provides ease of use.

The mouthpiece cover hiding the mouthpiece of the device pertaining to the present invention has been designed such that it also provides to actuate the device. Before each inhalation, both the mouthpiece is uncovered and one dose of the medicament in dry powder becomes ready for inhalation as one of the blister pockets is opened as a result of the mouthpiece cover's preferably being manually rotated along the path restricted by the engagement of the protrusions on the upper and the lower housing members. The rotational path on which the cover moves is restricted on both ends by the protrusions of the upper and the lower housing members. The constant-distance path that the protrusions of the upper and the lower housing members define results in the mouthpiece cover's being rotated by a fixed angle in response to the each actuation of the device.

The mouthpiece cover that triggers the gear mechanism of the device can be found solely in two positions. The mouthpiece cover can easily be switched from the first position to the second position with the help of the carved part in one end. When the mouthpiece cover is in the first position, the mouthpiece cover resides on the protrusion in one end of the rotational path. When the first position is on, the mouthpiece cover is completely covered and the device is in standby mode. When the mouthpiece cover is in the second position, the mouthpiece cover resides on the protrusion in the other end of the rotational path and one dose of the dry powder medicament becomes ready for inhalation upon the actuation of the device.

The mouthpiece cover of the device is joined with the gear mechanism via the connection points. One end of the drive gear passes through the center of the lower housing member and tightly joins with the mouthpiece cover in one connection point while the other end passes through the center of the upper housing member and joins with the mouthpiece cover in the other connection point. For each end of the drive gear to make a fixed connection between the connection points of the mouthpiece cover, one side cover is used for each end of the drive gear. The ends of the drive gear are carved for the ends of the side covers to be joined with each end of the drive gear from inside such that the ends of the side covers can tightly interlock with it. Thus, the drive gear is provided to be joined with the mouthpiece cover on both ends. The ends of the drive gear are carved such that they can tightly engage with the ends of the side covers from inside. The shape of the inside faces of these carved parts on both ends of the drive gear match with the shape of the ends of the side covers that fit into its. These covers that fit into each end of the drive gear pass through the connection point and enable the mouthpiece cover to synchronize with the drive gear.

On each connection point of the mouthpiece cover, there is a stabilizing resilient cover. The extensions under the stabilizing resilient covers pass through the holes on the upper or the lower housing members according to the position of the connection point and provide the stabilizing resilient covers that they connect with to remain stable. The rotation of the mouthpiece cover is prevented from both sides as the pawl under each of the stabilizing resilient covers in both sides of the device interlocks with the mouthpiece cover. Before the inhalation, the resilient parts of each stabilizing resilient cover that match with the shape of the fingers are pressed on for raising the pawls and releasing the mouthpiece cover in order to move the mouthpiece cover which actuates the device. Thus, the mouthpiece cover can easily be rotated when the resilient parts of each stabilizing resilient cover on both sides of the device that match with the shape of the fingers are pressed on. When the resilient parts of the stabilizing resilient covers are not pressed on, the pawls under the stabilizing resilient covers do not allow the mouthpiece cover to move in any circumstances.

The mouthpiece cover that triggers the gear mechanism of the device can be found solely in two positions. When the mouthpiece cover is in the first position, the mouthpiece cover resides on the protrusion in one end of the rotational path. When the first position is on, the mouthpiece cover is completely covered and the device is in standby mode. When the mouthpiece cover is in the second position, the mouthpiece cover resides on the protrusion in the other end of the rotational path and one dose of the dry powder medicament becomes ready for inhalation upon the actuation of the device.

According to the present invention, each gear of the gear mechanism in the device is directly or indirectly engages with each other. The drive gear, which is one of the components of the gear mechanism, provides the mouthpiece cover to trigger the gear mechanism. In each actuation of the device, the constant-angle rotational movement of the cover is transmitted by the indexing ratchet wheel which interlocks with the indexing wheel via the drive gear. The indexing wheel that synchronizes with the indexing ratchet wheel is engaged with the winding wheel gear and the pinion gear and causes them to move as well. Therefore, with the rotation of the indexing wheel, both the lid sheet of the blister package is provided to be coiled on the wings of the winding wheel as the winding wheel is rotated by the mechanism gear that engages with the winding wheel gear, and the counter gear that engages with the small gear under the base gear is provided to be rotated by means of the base gear that engages with the pinion gear.

According to the present invention, the indexing wheel is another component of the gear mechanism and it provides the blister package to be indexed properly and the opened blisters to be positioned accurately. The recesses of the indexing wheel match with the shape of the blister package and the blister pockets of the blister package are received in these recesses in sequence while the indexing wheel rotates. There are preferably 8 recesses on the indexing wheel. The angles between the recesses are all equal and 45°. The indexing wheel is supposed to rotate by the same angle in response to each actuation of the device for the opened blister pocket to be positioned accurately. As the indexing wheel rotates by the same angle, the blister package is indexed to the same extent and the opened blister pocket is provided to be accurately positioned.

At least one stopper component is arranged in any suitable part of the device in order to provide the blister package to be precisely positioned. The stopper can be in any suitable shape to execute said task.

The counter gear in the device pertaining to the present invention displays the number of the unused blister pockets remained in the device. In response to the actuation of the device by the mouthpiece cover, the mouthpiece is uncovered, the blister package is indexed and one dose of the dry powder medicament is prepared for inhalation while the counter gear rotates as well. Thus, the movement of the mouthpiece cover leads to the mouthpiece cover to be uncovered; one dose of the dry powder medicament to be ready for inhalation after the blister pocket is opened as well as providing the counter gear to rotate and display the new value of the unused blister pockets remained.

On the counter gear, there exist numerals equal to the number of the blister pockets present in the device and they are spaced by equal angles. In a device comprising 60 doses, the angle between the numerals is approximately 5°. The counter gear rotates as a result of the reflection of the rotation of the indexing wheel via the pinion gear and the base gear. In response to the each actuation of the device, rotation of the indexing wheel by the same angle each time due to the accurate transmission of the movement of the mouthpiece to the gear mechanism via the drive gear results in the rotation of the counter wheel approximately by the same angle as well and each numeral on the counter wheel is clearly seen through the display aperture on the upper housing member. Therefore, the patient makes sure about the number of the unused blister pockets remained in the device.

The inhaler according to the present invention further comprising a blister package composed of a plurality of blister pockets each of which comprises medicament in dry powder form and which are spaced at equal intervals. The blister package carries the medicament in dry powder form in one-dose portions and it is preferably a blister strip and it is preferably peelable. The blister pockets comprised in the blister package are spaced in equal intervals and each of them carries one dose of the medicament in dry powder form.

While the blister package is indexed on the indexing wheel, the beak on the housing peels the blister. Therefore, one dose dry powder medicament becomes ready for inhalation after the blister package is peeled to be opened in each actuation of the device.

The base sheet of the blister package on which the blister cavities are spaced is accumulated in the separated compartment of the housing. The lid sheet that provides impermeability of the blister package, on the other hand, is coiled on the winding wheel which is one of the components of the gear mechanism positioned in the other side of the housing.

The blister pocket opened as the blister package is peeled by the beak component of the inhaler is situated immediately under the manifold. Upon the inhalation of the patient, the airflow that preferably enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that also enable external air to enter the device easily and at convenient speed.

The mouthpiece is designed to fit the mouth for the patient to comfortably inhale the medicament in dry powder form. Depending on the shape of the device, the mouthpiece can be in any suitable shape or size as well as being fixed or movable. Furthermore, it can be attached or unattached to the upper and/or the lower cover.

The air inlet that the external air flow passes through is preferably designed not to be close where the patient holds the device in order not to prevent the air flow. Furthermore, in order to deliver the required amount of the dry powder medicament in the opened blister to the patient, the air inlet has been designed such that it allows the entry of the airflow through the air inlet by a convenient angle.

One end of the manifold communicates with the opened blister while the other end communicates with the mouthpiece. On the end of the manifold that communicates with the blister, at least two apertures with four sub-apertures are situated. Upon the inhalation of the patient, some of the air flow which enters through the air inlet passes through one of these apertures; entrains the medicament in dry powder form through the aperture to the manifold. The medicament in dry powder form that is entrained to the manifold with the airflow is delivered to the patient via the mouthpiece. The manifold can be in any appropriate shape while it is preferably longer than 1mm.

Each component of the device pertaining to the present invention can be made of any appropriate substance while it is preferably made of plastics. These plastic substances are selected from a group comprising styrene-acrylonitrile, polyoxymethylene (it is generally named as POM and it is also known as polyacetal or polyformaldehyde), acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer types while it is more preferably polyoxymthylene. The components made of plastics can be produced by methods such as injection molding. Furthermore, each component of the device can be in any appropriate color.

The lid and the base sheets constituting the blister package preferably consist of a plurality of layers. Each of these layers are preferably chosen from a group comprising polymeric layers that are made of various polymeric substances; aluminum foil and fluoropolymer film.

According to the present invention, the lid and base sheets composing the blister package are sealed very tightly by at least one of the methods comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive.

Fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

According to the invention, the thickness of the aluminum foil in the lid and the base sheets of the blister package are preferably chosen to be in the range of 5 to 80 µm, more preferably in the range of 15 to 65 µm.

According to the invention, the polymeric layers in the lid and the base sheets of the blister pack are made of the same or different polymers. The thickness of these polymeric layers varies according to the type of the polymeric substance used and its properties while they are preferably in the range of 5-100 µm, more preferably in the range of 15-60 µm.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be in the same or different shape, structure or volume.

The reference numbers of the drawings added to exemplify the present invention and the detailed description of the invention according to these drawings are given below but the scope of the invention should not be limited to these drawings.

### Brief Description of the Drawings

Figure 1 is a perspective view of an inhaler according to the inhaler described in the present invention;
Figure 2 is an exploded view of the inhaler pertaining to the invention;
Figure 3 is a perspective view of the blister pack for use with the inhaler pertaining to the invention;
Figures 4a and 4b are perspective views of the housing of the inhaler according to the invention;
Figures 5a and 5b are perspective views of upper and lower housing members of the inhaler according to the invention;
Figure 6a is a perspective view of the mouthpiece cover of the inhaler pertaining to the invention;
Figure 6b is an exploded view of the communication between the mouthpiece cover, the drive gear and the stabilizing resilient covers in the inhaler pertaining to the invention;
Figure 6c is a cross-sectional view of the communication between the mouthpiece cover, the drive gear and the stabilizing resilient covers in the inhaler pertaining to the invention;
Figure 6d is a cross-sectional view of the communication between the mouthpiece cover, the drive gear and the stabilizing resilient covers in the inhaler pertaining to the invention;
Figure 6e is an exploded view of the communication between the drive gear and the side covers in the inhaler pertaining to the invention;
Figure 6f is a cross-sectional view of the connection of the stabilizing resilient cover with the lower housing member in the inhaler pertaining to the invention;
Figures 7a-7c are cross-sectional views of the engagement of the gears composing the gear mechanism with each other in the inhaler pertaining to the present invention;
Figure 8 is a cross-sectional view of the blister package delaminating in course of operation of the inhaler pertaining to the present invention;
Figure 9 is a perspective view of the counter gear used in the inhaler pertaining to the present invention.

### Detailed Description of the Drawings

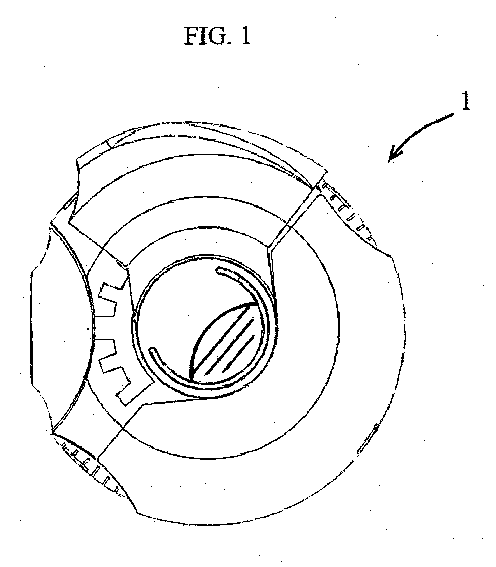

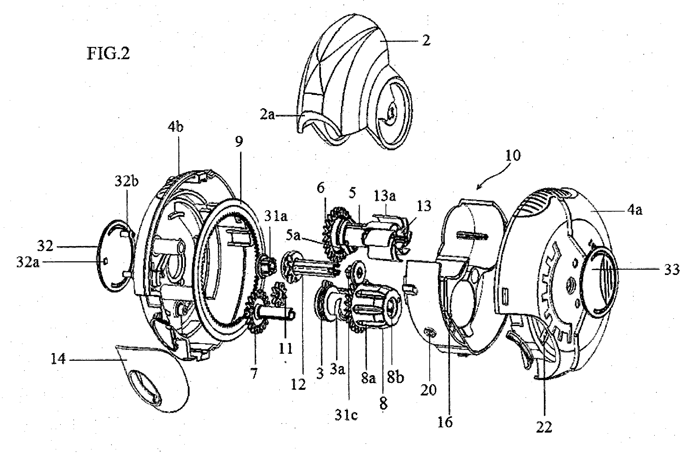

The inhaler (1) pertaining to the present invention comprises a gear mechanism situated in the housing (10) between the upper housing member (4a) and the lower housing member (4b) in order to enable the inhalation of the dry powder medicament carried in a blister package (15) as displayed in figures 1 and 2. Each component of the inhaler (1) is positioned at particular spots on the housing (10) to guarantee their working properly and accurately.

The inhaler (1) pertaining to the present invention shown in Figure 1 is ready for inhalation. In this case, the mouthpiece cover (2) is in the second position and the mouthpiece (14) is entirely exposed. The mouthpiece cover (2) has to be rotated by holding on the carved part (2a) on one end of the mouthpiece cover (2) in order to switch to the second position from the first position wherein the mouthpiece is completely covered. In this way, the mouthpiece (14) is completely exposed when the mouthpiece cover (2) is switched to the second position from the first position and the gear mechanism is triggered by the drive gear (12). The drive gear (12) precisely transmits the movement of the mouthpiece cover (2) to the indexing ratchet wheel (3).

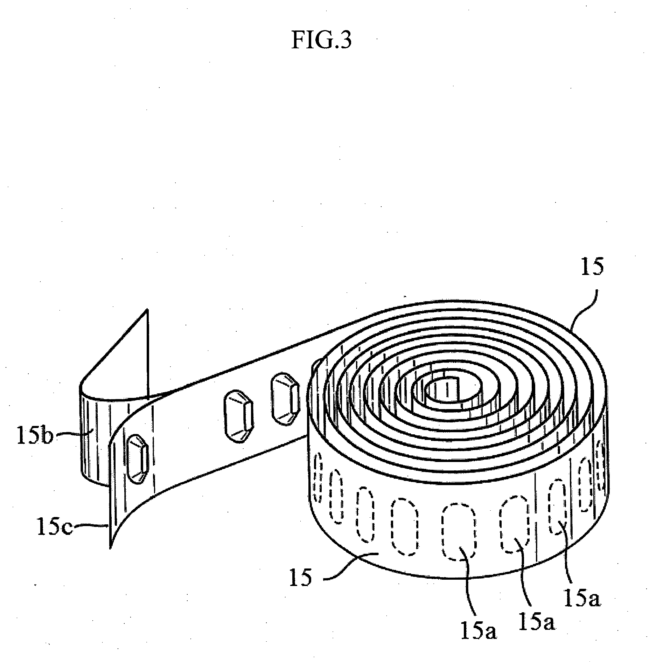

The indexing wheel (8) which engages with the indexing ratchet wheel (3) enables the blister package (15) shown in figure 3 to be indexed. The blister pockets (15a) composing the blister package are received in the recesses (8a) on the indexing wheel and the blister package (15) is indexed when the indexing wheel (8) rotates. In the inhaler pertaining to the present invention, shapes of the recesses (8a) on the indexing wheel (8) have been designed to match the shapes of the blister pockets (15) composing the blister package (15) for the blister package to be indexed properly.

The blister package (15) shown in figure 3 is composed of the lid sheet (15b) which provides impermeability and the base sheet (15c) on which the blister pockets (15a) are spaced at equal intervals. Each blister pocket contains medicament in dry powder form comprising one or more active agents.

The rotational movement that the mouthpiece cover (2) of the device executes while switching from the first position to the second is transmitted to the indexing ratchet wheel (3) via the drive gear (12) that the mouthpiece cover (2) engages with. As displayed in figure 2, arms (3a) of the indexing ratchet wheel interlocks with protrusions inside the indexing wheel (8) and rotates the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. The beak (16) in the housing (10) provides the blister package (15) to be peeled while the blister package (15) is indexed and provides one blister pocket (15a) to be opened in response to each actuation of the device (1).

The winding wheel gear (6), which is another component of the gear mechanism, engages with the indexing wheel (8) as displayed in figure 2. The mechanism gear (5) that interlocks the winding wheel (13) from inside has arms (5a) to interlock with the interior teeth of the winding wheel gear (6). When the indexing wheel (12) rotates the winding wheel gear (6), the winding wheel rotates unidirectionally owing to the arms of the mechanism gear (5a) which interlock with the interior teeth of the winding wheel gear (6) and the lid sheet (15b) which is peeled away while the blister package is indexed is tightly coiled on the wings (13a) of the winding wheel. The base sheet (15c) of the blister package (15) where the blister pockets are spaced is accumulated in a separate part (18a) of the device.

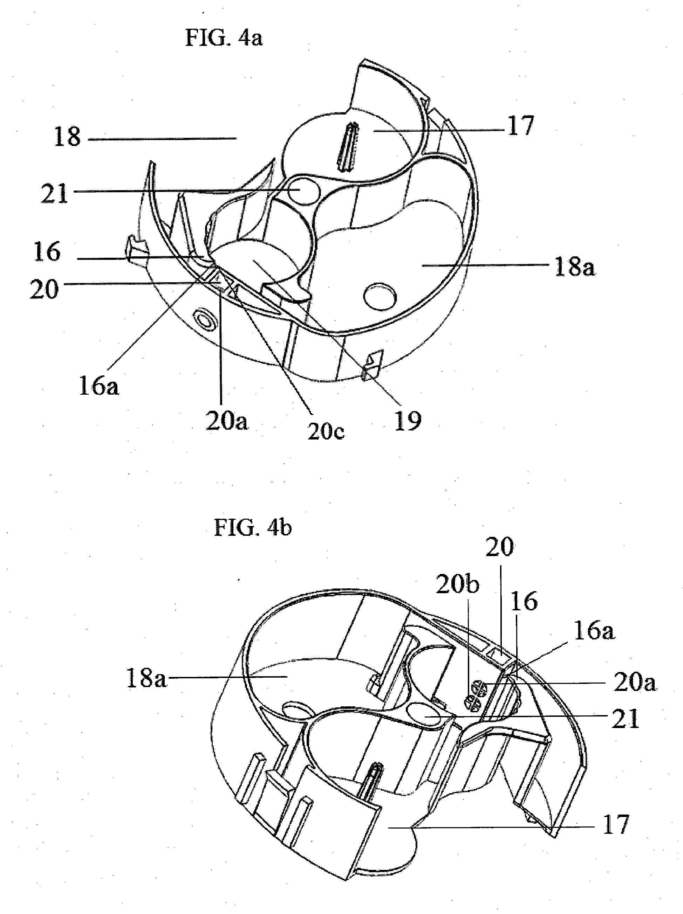

Different perspective views of the housing (10) wherein the gear mechanism and the other components of the inhaler (1) pertaining to the present invention are arranged are displayed in figures 4a and 4b. Furthermore, as can be seen in figures 4a and 4b, the housing (10) also comprises the other components having significant roles in the actuation of the device such as the beak (16), the manifold (20), the apertures with four sub-apertures (20a, 20b). Each component comprised in the housing is situated in appropriate parts of the housing (10) in order to enable the inhaler (1) to work properly. The drive gear (12) passes through the center (21) of the housing and joins the mouthpiece cover (2) at two points. The blister package (15) is in the lower part (17) of the housing as coiled up. In response to each actuation of the device (1), the blister package (15) is peeled by the beak (16) in the housing while being indexed by the indexing wheel (8) situated in the upper part (19) of the housing. The lid sheet (15b) of the blister package (15) which provides impermeability passes between the sharp point (20c) of the manifold and the sharp point of the beak (16a), is indexed over the beak (16) and coiled on the winding wheel (13) which is situated in the side part (18) of the housing. The base sheet (15c) of the blister package (15) on which the blister pockets (15a) are spaced, on the other hand, is accumulated in the separated compartment (18a) of the housing (10). Upon the inhalation of the patient, the air passes through the air inlet with four sub-apertures (20a) under the manifold (20) into the opened blister pocket; entrains the dry powder medicament contained in the opened blister pocket (15a) in response to each actuation of the device; provides it to pass through the other aperture with four-sub-apertures (20b) and reach the mouthpiece via the manifold (20).

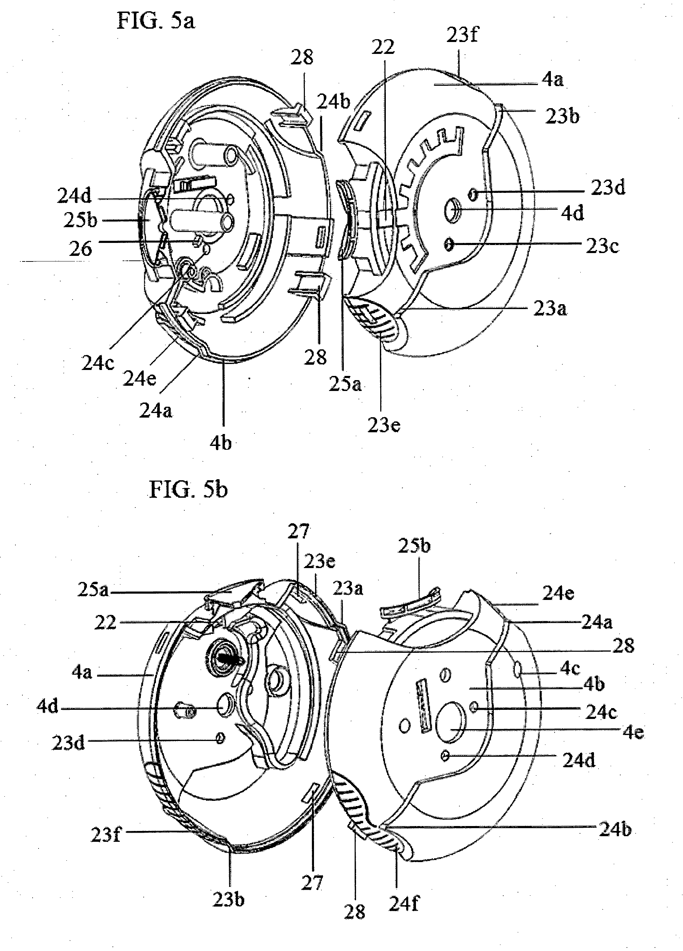

The housing (10) and the other components of the inhaler (1) pertaining to the present invention are stably kept together as the upper housing member (4a) and the lower housing member (4b) displayed in figures 5a and 5b are joined together. The engagement tabs (28) on the inside surface of the lower housing member (4b) engage with the engagement recesses (27) on the inside surface of the upper housing member (4a) and the upper and lower housing members are fixed tightly. Therefore, the protrusions (23a, 23b) on the upper housing member (4a) and the protrusions (24a, 24b) on the lower housing member (4b) are joined end to end and they define the restricted path for the rotational movement of the mouthpiece cover (2). The mouthpiece cover (2) can be moved along this path. When the mouthpiece cover (2) is in the first position, the mouthpiece is completely covered, the device is in standby mode and the mouthpiece cover (2) leans on the first protrusion (23a) on the upper housing member and the first protrusion (24a) on the lower housing member. The mouthpiece (2) is manually slid along the rotational path with the help of the carved part to switch to the second position. The mouthpiece is completely exposed when the cover is in this position, one dose of the dry powder medicament is ready for inhalation and the mouthpiece cover (2) leans on the second protrusion (23b) on the upper housing member and the second protrusion (24b) on the lower housing member.

As displayed in figures 5a and 5b, one half (25a) of the tapered channel that interconnects the manifold (20) that exist in the housing (10) with the mouthpiece (14) is comprised in the upper housing member (4a) while the other half of it (25b) is comprised in the lower housing member (4b). The channel is constituted as a whole when the upper (4a) and the lower (4b) housing members are joined together. Upon the inhalation of the patient, the air that enters the device through the air inlet (22) arranged in the upper housing member (4a) passes through the aperture with four sub-apertures (20a), reaches the opened blister (15a) and entrains the dry powder medicament there to the manifold (20) by passing it through the other aperture with four sub-apertures (20b). The grids on the upper housing member (23e, 23f) and the grids on the lower housing member (24e, 24f) prevent the slips of fingers when rotating the mouthpiece cover.

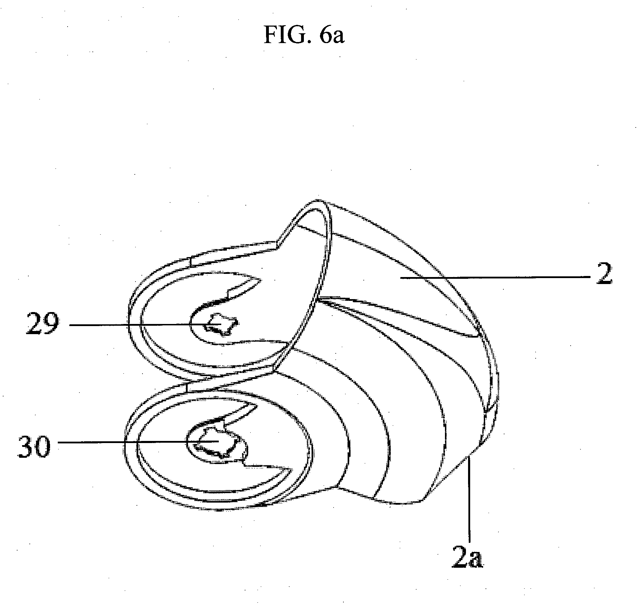

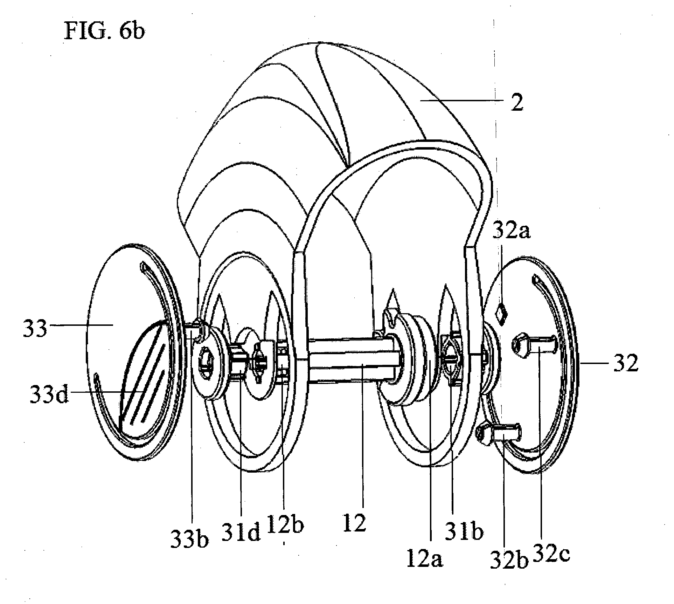

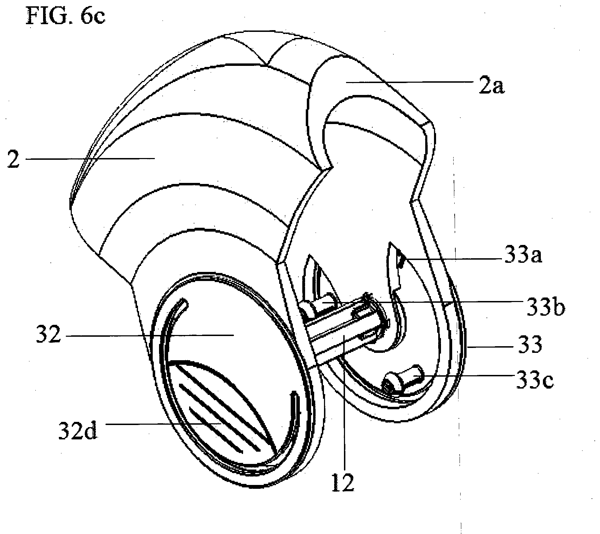

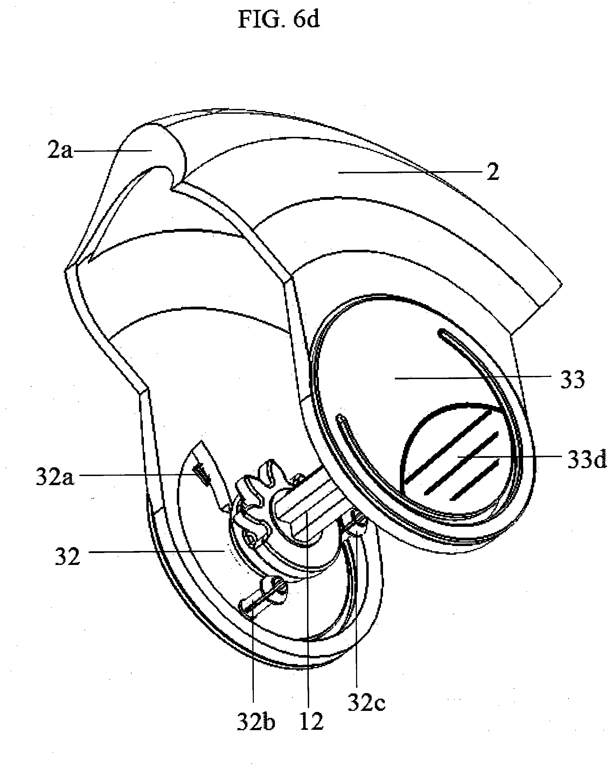

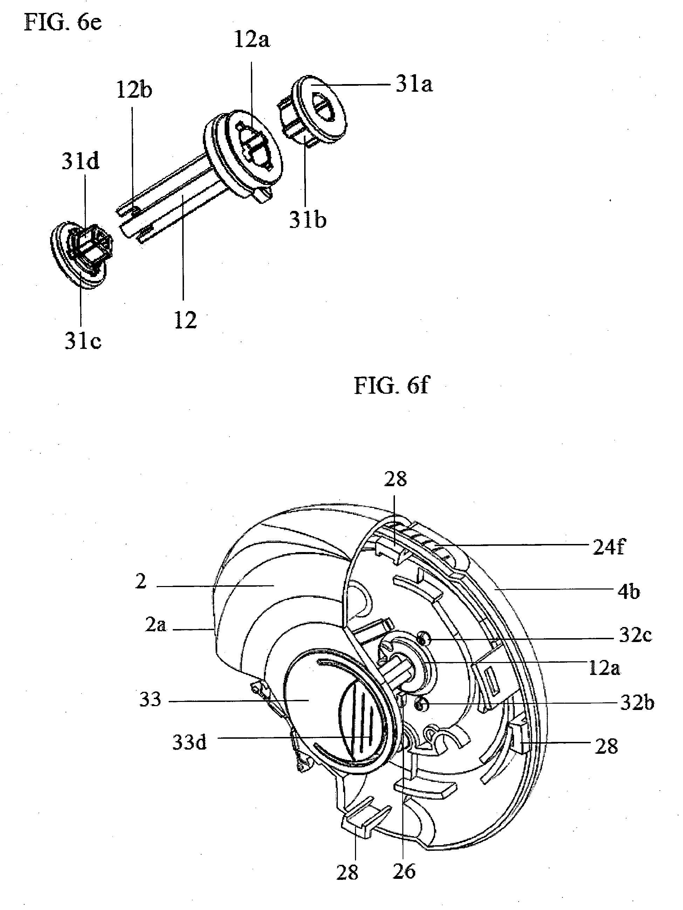

The mouthpiece cover (2) of the inhaler pertaining to the present invention is displayed in figure 6a. The carved part (2a) in one end of the device enables to easily move the mouthpiece cover manually. The mouthpiece cover (2) is joined with the gear mechanism via the connection points. The drive gear (12) is joined with the connection points (29, 30) of the mouthpiece cover via the side covers (31 a, 31 c) as it can clearly be seen in figures 6b, 6c and 6d illustrating the communication between the mouthpiece cover (2), the drive gear (12), side covers (31a, 31c) and the stabilizing resilient covers (32,33). Each of these side covers (31a; 31c) passes through the center (4d) of the upper housing member or the center (4e) of the lower housing member and joins with the end (12a; 12b) of the drive gear. It can clearly be seen in figure 6d that the both ends (12a; 12b) of the drive gear is carved such that the end of the side cover (31b; 31 d) can pass through. Each end of the side covers (31d; 31b) passes through one of the connection points (29; 30) of the mouthpiece cover and it is received in the recess in one end (12b; 12a) of the drive gear, thus it provides to tightly and stably interconnect the mouthpiece cover (2) with the drive gear (12). It is provided that the mouthpiece cover (2) synchronizes with the drive gear (12) as the connection point (29; 30) of the mouthpiece cover which has a matching shape with the ends (31d; 31b) of the side covers that passes through it on both sides of the device and the end (12b; 12d) of the drive gear that it communicates with are on the same component.

As is seen from figures 6a-6e, the shapes of the ends (31b; 31d) of the side covers that are received in the carved parts on the ends of the drive gear and the shapes of the connection points (29, 30) of the mouthpiece cover are not identical since the two ends (12a, 12b) of the drive gear are not identical.

There is one stabilizing resilient cover (33; 32) on each connection point (29; 30) of the mouthpiece and on each side cover, as displayed in figures 2, 6a-6d and 6f. When the mouthpiece cover (2) is in the first position, the pawls (32a, 33a) under the stabilizing resilient covers, which are on the connection points (29, 30) of the mouthpiece, interlock with the mouthpiece cover (2) on both sides as clearly seen in figures 6c and 6d. The pawl (33a) under the stabilizing resilient cover that is on the first connection point (29) interlocks with the mouthpiece cover on one side (figure 6c). Identically, the pawl (32a) under the stabilizing resilient cover that is on the second connection point (30) of the mouthpiece cover interlocks with the mouthpiece cover (2) on the other side (figure 6d). Thus, these pawls (32a, 33a) under the stabilizing resilient covers prevent the movement of the mouthpiece cover (2) by interlocking with it on both sides, and therefore the inadvertent actuation of the drive mechanism.

The extensions (32b, 32c; 33b, 33c) under the stabilizing resilient covers pass through the apertures (23c, 23d; 24c, 24d) on the upper and the lower housing members illustrated in figures 5a and 5b and provide the stabilizing resilient covers to remain stable. Namely, the extensions (33b; 33c) under the stabilizing resilient cover that is on the first connection point (29) of the mouthpiece cover pass through the apertures (23c; 23d) on the upper housing member and provide the stabilizing resilient cover (33) to be stably joined with the device. Identically, the extensions (32b, 32c) under the stabilizing resilient cover on the second connection point (30) of the mouthpiece cover pass through the apertures (24c, 24d) on the lower housing member and provide the stabilizing resilient cover (32) to be stably joined with the device as clearly illustrated in figure 6f.

Before the inhalation, the resilient parts (32d, 33d) of each stabilizing resilient cover illustrated in figures 6c and 6d are pressed on for raising the pawls (32a, 33a) and releasing the mouthpiece cover (2) in order to actuate the gear mechanism of the device to prepare one dose of dry powder medicament before inhalation. Therefore, the gear mechanism of the device is actuated and one blister pocket (15a) is opened for one dose of the dry powder medicament to be ready for inhalation when the resilient parts (32d, 33d) of the stabilizing resilient covers are pressed on and the mouthpiece cover (2) is switched from the first position to the second position simultaneously. The necessity to press on the resilient parts (32d, 33d) of the stabilizing resilient covers so as to actuate the gear mechanism preclude the consequences which may result from accidental and inadvertent actuations of the gear mechanism.

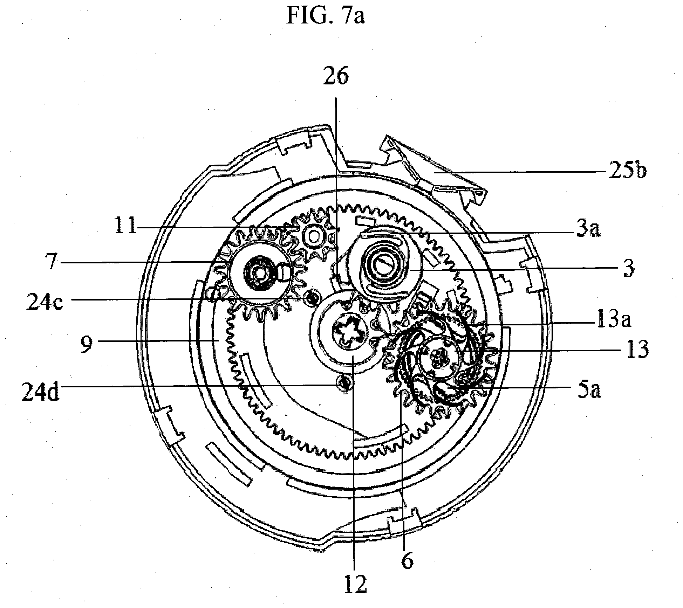

In figure 7a, it is displayed that the stopper (26) interlocks with the tooth of the indexing ratchet wheel (3) and hinders its rotation. The rotational movement of the mouthpiece cover (2) by the same angle in response to each actuation of the device (1) is accurately transmitted to the indexing ratchet wheel (3) by the drive gear (12) which engages with the mouthpiece cover (2) on its both ends and the drive gear (12) is enabled to rotate by the same angle in each actuation of the device (1). The stopper component (26) in the lower housing member (4b) prevents the backwards movement of the blister package (15) indexed by the indexing wheel (8) which synchronizes with the indexing ratchet wheel by keeping the position of the indexing ratchet wheel (3) fixed and provides the blister package (15) to be precisely positioned.

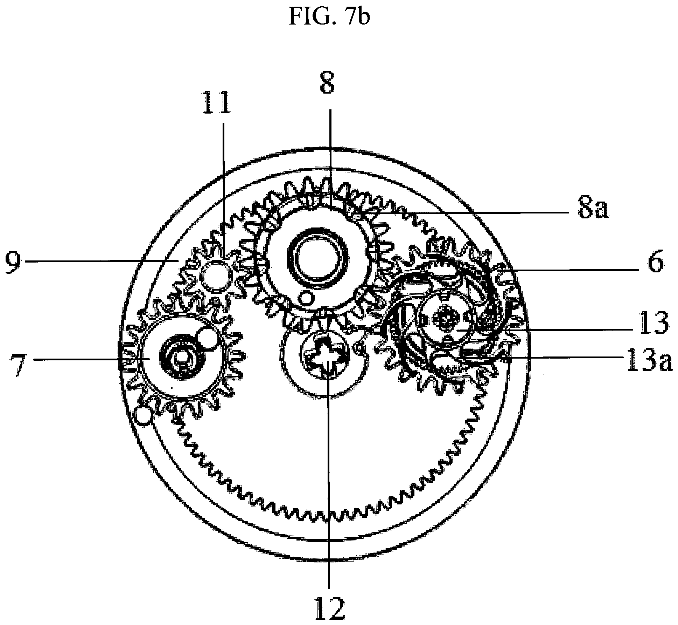

As can be seen in figure 7b, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15) which is indexed by the rotation of the indexing wheel (8) is tightly coiled on the winding wheel (13) engaging with the winding wheel gear (6) and also the counter wheel (9) is provided to be moved by the pinion gear (11) and the base gear (7) as a result of the rotation of the indexing wheel (8).

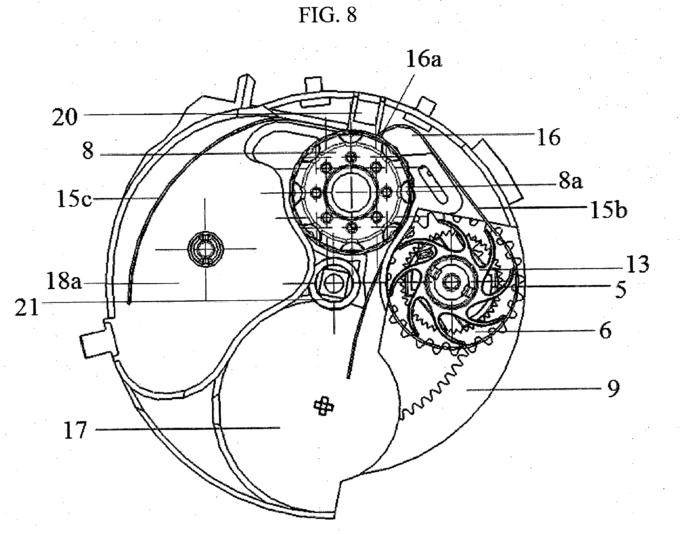

As is seen in figure 8, the lid sheet (15b) of the blister package (15) which is peeled away by the beak (16) and the base sheet (15c) are enclosed in separate compartments of the housing (10). The lid sheet (15b) that provides impermeability is indexed over the beak (16) and tightly coiled on the wings (13a) of the winding wheel. The base sheet (15c) of the blister package (15) where the blister pockets (15a) each of which carries one dose of the dry powder medicament are spaced is accumulated in the separated compartment (18a) of the housing (10). In response to each actuation of the device (1), one dose of the dry powder medicament which is prepared for inhalation after one blister pocket (15a) is opened and the air entering the device through the air inlet (22) upon the inhalation of the patient provides to deliver one dose of the dry powder medicament to the patient by entraining it from the blister pocket (15a) to the mouthpiece (14).

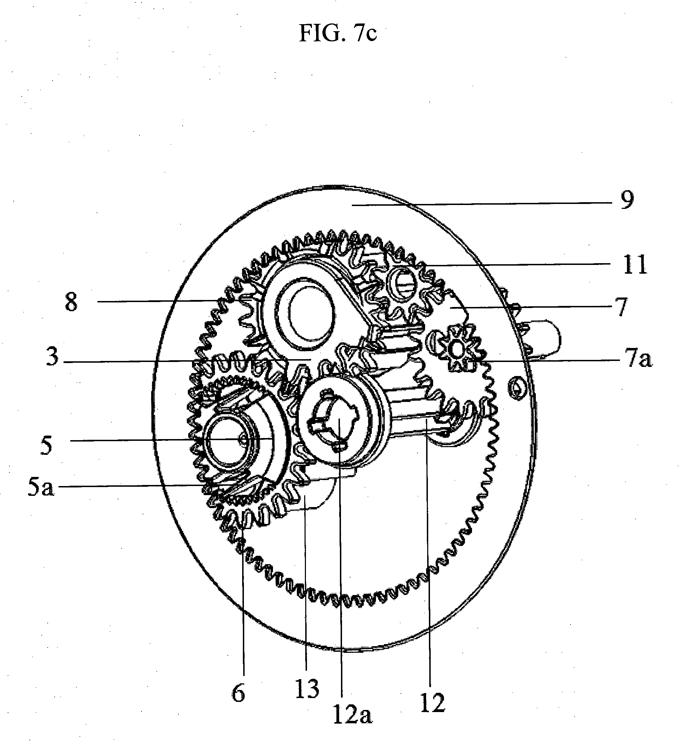

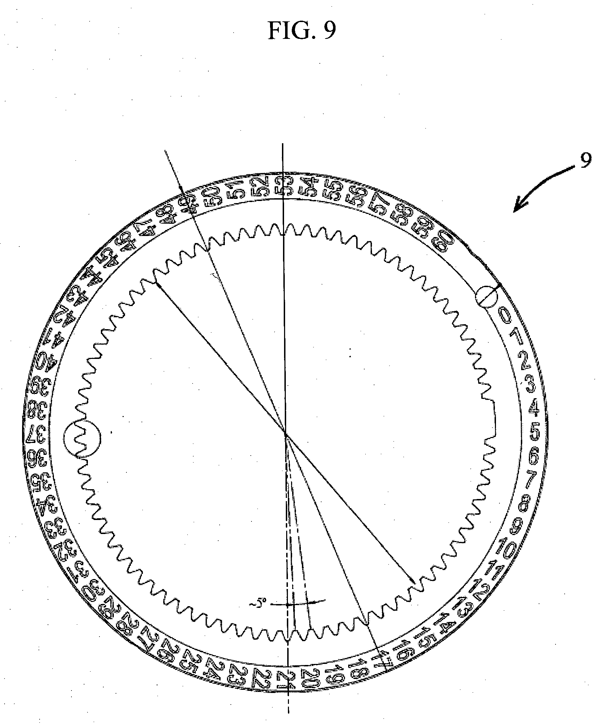

The rotation of the indexing wheel (8) is transmitted to the base gear (7) engaging with the pinion gear (11) by the pinion gear (11). The small gear which is under the base gear (7) as attached to it engages with the counter gear (9) (figure 7c). Thus, the movement of the indexing wheel (8) is transmitted to the counter wheel (9) shown in figure 9 by the pinion gear (11) and the base gear. There are numerals incrementing from 1 to 60 in the counter gear (9) displayed in figure 9. The angles between these numerals are all equal and approximately 5°. In response to each actuation of the device, the counter gear rotates approximately 5° and the number of the unused blister pockets remained in the device are clearly seen through the display aperture (4c) on the lower housing member (4b).

In use of the device described in figures 1-9, the mouthpiece (14) is exposed when the mouthpiece cover (2) is slid from the first position to the second on the upper housing member (4a) and the lower housing member (4b); the gear mechanism is triggered by the drive gear (12) and one dose of dry powder medicament is prepared for inhalation; the counter gear (9) is indexed and the numeral seen through the display aperture (4c) on the lower housing member (4b) is incremented. After the inhalation is realized, the mouthpiece cover (2) is solely moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhaler pertaining to the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device pertaining to the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, steroids, antiinflammatories, bronchodilators, leukotirene inhibitors, PDE IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in the administration of the medicament in dry powder form which is utilized in the treatment of many respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention.

## Claims

1. An inhaler suitable for delivery of the medicament in dry powder form from a blister package (15) composed of a plurality of blister pockets (15a) each of which comprises medicament in dry powder form and which are spaced at equal intervals; said inhaler comprising:
- a beak (16) which provides to peel a blister package (15),
- a mouthpiece (14) enabling the patient to inhale the medicament in dry powder form an opened blister;
- a rotatable mouthpiece cover (2) covering the mouthpiece (14);
- a gear mechanism enabling a blister package (15) to be indexed and the medicament in dry powder form to become ready for inhalation;
- a manifold (20) with an end in communication with the opened blister and an opposing end in communication with the mouthpiece thereby being located between the mouthpiece and the opened blister;
- a housing (10) situated between the upper housing member (4a) and the lower housing member (4b) in which a blister package and the gear mechanism are enclosed **characterized in that** the radius of the sharp point (16a) of the beak (16) that is in contact with the lid sheet (15b) of a blister package (15) indexed upon the actuation of the device is in the range of 0.05 to 1 mm, and
wherein the manifold has a sharp point (20c), the distance between the sharp point of the manifold and the sharp point (16a) of the beak being in the range of 0.005 mm to 0.5 mm.

2. The inhaler (1) according to claim 1 **characterized in that** the radius of sharp point (16a) of the beak (16) that is in contact with the lid sheet (15b) of the blister pack is in the range of 0.05 mm to 0.4 mm.

3. The inhaler (1) according to claim 1, wherein the beak (16) and the manifold (20) are accommodated in the housing (10) of the inhaler.

4. The inhaler (1) according to claim 1, wherein a restricted path is arranged with the protrusions (23a, 23b; 24a, 24b) on the upper and lower housing members.

5. The inhaler (1) according to claim 1, wherein the mouthpiece cover (2) is rotationally moved by being slid on the upper (4a) and the lower (4b) housing member.

6. The inhaler (1) according to any one of the preceding claims, wherein the mouthpiece cover (2) can solely be in two positions:
- the mouthpiece (14) is completely covered and the device (1) is on standby mode when the mouthpiece cover (2) is in the first position,
- one dose of the medicament in dry powder form is ready for inhalation upon the actuation of the device (1) when the mouthpiece cover (2) is in the second position.

7. The inhaler (1) according to claim 1, wherein there is a carved part (2a) in one end of the mouthpiece cover so as to rotate the mouthpiece cover easily.

8. The inhaler (1) according to claim 1, wherein all components of the gear mechanism is directly or indirectly engages with each other.

9. The inhaler (1) according to claim 1 or 8, wherein said gear mechanism is composed of;
- the drive gear (12) which provides to actuate the device (1) by transmitting the constant angle movement of the mouthpiece cover (2) to the indexing ratchet wheel (3);
- the indexing wheel (8) which synchronizes with the indexing ratchet wheel (3) and enables the blister package (15) to be indexed;
- the winding wheel gear (6) which moves the winding wheel (13) via the mechanism wheel (5) upon the rotation of the indexing wheel (8);
- the pinion gear (11) and the base gear (7) that provide to transmit the movement of the indexing wheel (8) to the counter wheel (9);
- the counter wheel (9) which displays the number of the unused blister pockets (15a) remained in the device (1).

10. The inhaler according to any of the proceeding claims, further comprising a blister package (15) composed of a plurality of blister pockets (15a) each of which comprises medicament in dry powder form and which are spaced at equal intervals.

11. The inhaler (1) appropriate for delivery of the medicament in dry powder form according to any one of the preceding claims, wherein said medicament in dry powder form comprises at least one active agent selected from a group comprising cromolyns, anti-infectives, antihistamines, steroids, anti-inflammatories, bronchodilators, leukotirene inhibitors, PED IV inhibitors, antitussives, diuretics, anticholinergics, hormones, xanthines and pharmaceutically acceptable combinations thereof.

## Patentansprüche

1. Mit mehreren gleichabständen angeordneten Blistergefäße (15a), welches je Trockenpulver in Arzneiform enthalten, besteht aus einer Blisterverpackung (15) mit einer Inhalation für die Übertragung des Medikaments, welche Inhalation die unten angegeben Punkte umfassen:
- eine Blisterverpackung (15) ist mit einem Ansatz (16) für die Ermöglichung des herausgleiten ausgestattet.
- die Düse (14) erlaubt den Patienten das Medikament Trückenpulver durch die Blisteröffnung zu inhalieren.
- Ein drehbarer überziehender Düsendeckel (2) für die Düse (14);
- Blisterpackung (15) und für die sofortige Inhalieren des Medikament Trockenpulver ein markierbares Zahnradmechanismus
- Die geöffnete Blister steht in Verbindung zum Inhalier-Ansatz, welches als Verteiler (20) dient; In der Blisterverpackung und Zahnradmechanismus ein Obergefäß (4a) und ein Gefäß (10),welches sich zwischen den Untergefäßelement (4b)befindet;
Mit der Aktivierung des gekennzeichneten Blisterverpackung Geräts, **dadurch gekennzeichnet, dass** das Blisterverpackung (15) Deckelfolie (15b) in Verbindung zur Ansatzöffnungs- (16) Spitze (16a) den Radius von 0.05-1mm hat und der Verteiler eine Spitze (20c) hat, welches Abstand von der Ansatzspitze (16a) und Verteilerspitze 0.005mm -0.5mm beträgt.

2. Inhalierter (1) nach Anspruch 1, **dadurch gekennzeichnet, dass** die Deckelfolie der Blisterverpackung (15) in Verbindung zur Ansatz- (16) Spitze (16a) mit einem Radius 0.05mm - 0.4mm steht.

3. Inhalierter (1) Nach Anspruch 1, wobei das Inhalierter Gefäß (10) Ansatz (16) und Verteiler (20) beinhaltet.

4. Inhalierter (1) Nach Anspruch 1, wobei es eine begrenzte Ober- und Untergefäßelemente mit Ansätze (23a, 23b; 24a, 24b) verfügt.

5. Inhalierter (1) Nach Anspruch 1, wobei Mundstückabdeckung (2) gleitend und drehend auf (4a) und unter (4b) der Gefäßelemente.

6. Inhalierter (1) nach einer der vorgenannten Ansprüche, wobei der Mundstückabdeckung (2) ausschließlich in zwei Positionen sein kann:
- Das Mundstück (14) vollständig abgedeckt ist und die Vorrichtung (1) im Standby-Modus ist während die Mundstückabdeckung (2) in der ersten Position ist;
- Mundstückabdeckung (2) bei der zweiten Position ist im Aktiv -Modus und das Gerät (1) bereit das Medikament Trockenpulver zu inhalieren.

7. Inhalierter (1) Nach Anspruch 1, wobei sich am Mundstückabdeckung ein einfach drehbarer Ansatzschlitz (2a) an der Spitze befindet;

8. Inhalierter Nach Anspruch 1, wobei der Zahnradmechanismus mit allen Komponenten direkt und indirekt im Zusammenarbeit ist.

9. Inhalierter (1) Nach Anspruch 1 oder 8, wobei das Zahnradmechanismus aus folgendem besteht;
- Für die Markierung durch das Sperrrad (3) wird der Düsendeckel (2) mit dem Überleiten einer Winkelbewegung das Gerät (1) durch den Antriebszahnrad aktiviert (12).
- Das markierbare Sperrrad (3), welches mit der Blisterverpackung (15) synchronisiert ist, ist mit einem Kennzeichnungs-Rad (8)
- Die Drehung des Laufmechanismus (5) bringt das Kennzeichnungs-Rad (8) den Winkelrad (13) und somit das Winkelzahnrad in Bewegung (6);
- Das Zählrad (9) wird mit der Bewegung des Kennzeichnungs-Rad (8) an den Basiszahnrad (7) und Ritzel weitergeleitet (11).
- Im Gerät (1) befindet sich für die benutzten Blisterverpackungengefässe (15a) ein Zählrad(9).

10. Inhalierter Nach einem der vorgenannten Ansprüch, ferner umfassend eine Blisterverpackung (15), die aus einer Vielzahl von Blistertaschen (15a) besteht, die jeweils ein Medikament in Trockenpulverform umfassen und die in gleichen Abständen beabstandet sind.

11. Inhalierter (1), der für die Abgabe des Medikaments in Trackenpulverform gemäß einem der vorgenannten Ansprüche geeignet ist, wobei das Medikament in Trockenpulverform mindestens einen Wirkstoff, ausgewählt aus einer Gruppe, umfassend Cromolyn, Antiinfektiva, anthistam, Steroide, entzündungshemmende Mittel, Bronchodilatatoren, Leukotrien-Inhibitoren, PED IV Inhibitoren, Antitussiva, Diuretika, Anticholinergika, Hormone, Xanthine zusammengestezt und pharmazeutischen verträgliche Kombinationen davon.

## Revendications

1. Un inhalateur approprié pour l'administration du médicament en forme de poudre sèche d'un emballage-coque (15) constitué de plusieurs poches-coque (15a), dont chacune comporte le médicament en forme de poudre sèche et est espacée à des intervalles égaux; ledit inhalateur comportant:
- un gobelet (16) permettant de décoller un emballage-coque (15),
- un embout buccal (14) permettant au malade d'inhaler le médicament en forme de poudre sèche d'une cloque ouverte;
- un couvercle d'embout buccal pivotant (2) couvrant l'embout buccal (14);
- un mécanisme d'engrenage permettant l'indexage de l'emballage-coque (15) et au médicament en forme de poudre sèche de devenir prêt l'inhalation;
- un collecteur (20)
avec une extrémité en communication avec la cloque ouverte et une extrémité opposée en communication avec l'embout buccal ainsi étant située entre l'embout buccal et la cloque ouverte;
- un logement (10) situé entre le membre de logement supérieur (4a) et le membre de logement inférieur (4b) dans lequel un emballage-coque et un mécanisme d'engrenage sont enfermés, **caractérisé en ce que** le rayon du point aigu (16a) du gobelet (16) que est en contact avec la plaque de couvercle (15b) d'un emballage-coque (15) indexé sur l'activation du dispositif est dans la plage de 0,05 à 1 mm, et
où le collecteur possède un point aigu (20c), la distance entre le point aigu du collecteur et le point aigu (16a) du gobelet étant dans la plage de 0,005 mm à 0,5 mm.

2. L'inhalateur (1) selon La revendication 1, **caractérisé en ce que** le rayon du point aigu (16a) du gobelet (16) qui est en contact avec la plaque de couvercle (15b) de l'emballage-coque est dans la plage de 0,05 mm à 0,4 mm.

3. L'inhalateur (1) selon la revendication 1, dans lequel le gobelet (16) et le collecteur (20) sont logés dans le logement (10) de l'inhalateur.

4. L'inhalateur (1) selon la revendication 1, dans lequel une voie restreinte est arrangée avec les saillies (23a, 23b; 24a, 24b) sur les membres de logement supérieurs et inférieurs.

5. L'inhalateur (1) selon la revendication (1), dans lequel le couvercle d'embout buccal (2) se déplace dans une manière rotative en étant glissé sur les membres de logement supérieurs (4a) et inférieurs (4b).

6. L'inhalateur (1) selon l'une quelconque des revendications précédentes, dans lequel le couvercle d'embout buccal (2) peut être seulement dans deux positions:
- l'embout buccal (14) est complètement recouvert et le dispositif (1) est dans le mode veille quand le couvercle d'embout buccal (2) est dans la première position,
- une dose du médicament en forme de poudre sèche est prête à l'inhalation sur l'activation du dispositif (1) quand le couvercle d'embout buccal (2) est dans la deuxième position.

7. L'inhalateur (1) selon la revendication 1, dans lequel il y a une partie sculptée (2a) dans une extrémité du couvercle d'embout buccal afin de pivoter le couvercle d'embout buccal facilement.

8. L'inhalateur (1) selon la revendication 1, dans lequel tous les composants du mécanisme d'engrenage se sont engagés les uns avec les autres directement ou indirectement.

9. L'inhalateur (1) selon la revendication 1 ou 8, dans lequel ledit mécanisme d'engrenage est constitué de.
- le pignon de commande (12) assurant l'activation du dispositif (1) en transmettant le mouvement d'angle constant du couvercle d'embout buccal (2) à la roue à rochet d'indexage (3);
- la roue d'indexage (8) synchronisant avec la roue à rochet d'indexage (3) et permettant à l'emballage-coque (15) d'être indexé;
- la roue de bobinage d'engrenage (6) déplaçant la roue de bobinage (13) via la roue de mécanisme (5) sur la rotation de la roue d'indexage (8);
- l'engrenage à pignons (11) et l'engrenage de base (7) permettant à la transmission du mouvement de la roue d'indexage (8) de la roue compteur (9);
- la roue compteur (9) affichant le nombre des poches-coque (15a) restant dans le dispositif (1).

10. Un inhalateur selon l'une quelconque des revendications précédentes, comportant en outre un emballage-coque (15) constitué de plusieurs poches-coque (15a), dont chacune comporte le médicament en forme de poudre sèche et est espacée à des intervalles égaux.

11. L'inhalateur (1) approprié pour l'administration du médicament en forme de poudre sèche selon l'une quelconque des revendications précédentes, dans lequel ledit médicament en forme de poudre sèche comporte au moins un principe actif choisi parmi un groupe comportant le cromolyn, les anti-infectieux, les anti-histamines, les stéroïdes, les anti-inflammatoires, les bronchodilateurs, les inhibiteurs leucotriènes, les inhibiteurs de PED IV, les anti-tussifs, les diurétiques, les anticholinergiques, les hormones, les xanthines, et leurs combinaisons pharmaceutiquement acceptables.
